# EUROPÄISCHE PATENTSCHRIFT

(11) **EP 4 448 047 B1**
(45) Veröffentlichungstag und Bekanntmachung des Hinweises auf die Patenterteilung: **28.10.2026**
(21) Anmeldenummer: 22838718.9
(22) Anmeldetag: 14.12.2022
(51) Int. Cl.: A61M 1/36, A61M 1/16, A61M 1/34

(54) **BLUTBEHANDLUNGSVORRICHTUNG MIT HEIZUNGSREGELUNG**
BLOOD TREATMENT DEVICE WITH HEATING CONTROL
DISPOSITIF DE TRAITEMENT DU SANG AVEC COMMANDE DE CHAUFFAGE

(30) Priorität: 17.12.2021 DE 102021133630
(43) Veröffentlichungstag der Anmeldung: 23.10.2024
(73) Patentinhaber: Fresenius Medical Care Deutschland GmbH, 61352 Bad Homburg (DE)
(72) Erfinder: BREUEL, Lars, 61352 Bad Homburg (DE); HELBIG, Frank, 61352 Bad Homburg (DE); JAKOB, Eckhard, 61352 Bad Homburg (DE)
(74) Vertreter: Bobbert & Partner Patentanwälte PartmbB
(86) Internationale Anmeldenummer: PCT/EP2022/085799
(87) Internationale Veröffentlichungsnummer: WO 2023/110986

(56) Entgegenhaltungen:
- US-A1- 2014 199 193
- US-A1- 2019 160 216
- US-A1- 2020 282 126

## Beschreibung

Die vorliegende Erfindung betrifft eine Blutbehandlungsvorrichtung gemäß Anspruch 1.

Aus der Praxis sind Blutbehandlungsvorrichtungen bekannt, welche mittels einer Pumpe eine Behandlungsflüssigkeit dem Patienten applizieren, oft, wie im Beispiel der Dialysevorrichtung, über den extrakorporalen Blutkreislauf. Bei Blutbehandlungsvorrichtungen wie etwa Dialysevorrichtungen, wird Behandlungsflüssigkeit ergänzend oder alternativ z. B. einem blutdurchströmten Blutfilter zugeführt. Da die Temperatur der geförderten Behandlungsflüssigkeit in beiden vorgenannten Fällen auf die Temperatur des dem Patienten zu reinfundierenden Blutes Einfluss haben kann, kann eine Heizvorrichtung zum Erwärmen oder Temperieren der Behandlungsflüssigkeit vorgesehen sein. US 2014/199193 A1 beschreibt ein Hämodialysesystem mit Dialysatkreislauf, Kassette, Heizer und Temperatursensor. Ein Fluid-Temp-Control-Modus reduziert den Dialysatfluss temporär, wenn der Heizer seine Obergrenze erreicht, und erhöht ihn später wieder. Steuerparameter wie Integratorwerte und vorherige Flussraten werden gespeichert.

US 2019/160216 A1 betrifft eine Blutbehandlungsvorrichtung mit Rezirkulationskreislauf, Pumpe, Heizer und Temperaturmessung am Blutrücklauf. Die Regelung passt die Erwärmungseinrichtung oder die Rezirkulationsflussrate an, um einen Bluttemperatur-Sollwert zu erreichen, unter Berücksichtigung stromabwärtiger Wärmeverluste.

Eine Aufgabe der vorliegenden Erfindung ist es, eine weitere Blutbehandlungsvorrichtung mit einer Heizvorrichtung anzugeben. Ferner wird eine Steuervorrichtung für eine solche Blutbehandlungsvorrichtung beschrieben, die nicht zum Gegenstand der in den Ansprüchen definierten Erfindung gehört.

Ferner sollen ein digitales Speichermedium, ein Computerprogramm-Produkt und ein Computerprogramm angegeben werden.

Die erfindungsgemäße Aufgabe wird durch eine Blutbehandlungsvorrichtung mit den Merkmalen des Anspruchs 1 gelöst. Ferner werden im Folgenden eine Steuervorrichtung, ein digitales Speichermedium, ein Computerprogramm-Produkt sowie ein Computerprogramm beschrieben, die nicht zum Gegenstand der in den Ansprüchen definierten Erfindung gehören.

Erfindungsgemäß wird eine Blutbehandlungsvorrichtung vorgeschlagen, welche eine optionale Blutpumpe, wenigstens eine Fluidleitung, eine Pumpe, konfiguriert zum Fördern von Behandlungsflüssigkeit durch die Fluidleitung hindurch und wenigstens eine Heizvorrichtung zum Erwärmen der Behandlungsflüssigkeit innerhalb der Fluidleitung und/oder innerhalb der Heizvorrichtung, aufweist oder hiermit verbunden ist. Alternativ weist die Blutbehandlungsvorrichtung eine Verbindungsstelle zu ihrem Verbinden mit einer Fluidleitung, wie hierin ausgeführt, auf. Ferner weist die erfindungsgemäße Blutbehandlungsvorrichtung eine Temperaturmessvorrichtung auf, welche zum Erheben der Temperatur der Behandlungsflüssigkeit, wenn diese in der Fluidleitung oder in der Heizvorrichtung vorliegt, vorgesehen und/oder konfiguriert ist. Das Erheben kann beispielsweise ein Messen oder Ermitteln, der Temperatur der Behandlungsflüssigkeit mittels wenigstens eines Temperatursensors sein oder umfassen.

Eine Speichervorrichtung zum Speichern von Beträgen von Temperaturwerten ist ebenfalls von der Blutbehandlungsvorrichtung umfasst. Die Beträge der Temperaturwerte können die mittels der Temperaturmessvorrichtung erhobenen Werte sein oder umfassen.

Die erfindungsgemäße Blutbehandlungsvorrichtung umfasst weiter eine Steuervorrichtung, hierin auch als erfindungsgemäße Steuervorrichtung bezeichnet, zum Steuern oder Regeln der vorstehend genannten Pumpen, insbesondere der Pumpe, welche zum Fördern von Behandlungsflüssigkeit konfiguriert ist. Optional ist die Steuervorrichtung ferner zum Steuern oder Regeln der wenigstens einen Heizvorrichtung konfiguriert.

Die Steuervorrichtung ist weiter programmiert oder konfiguriert, um die Temperaturmessvorrichtung zu veranlassen, zu einer Vielzahl von Messzeitpunkten, welche verschiedenen Messintervallen zugeordnet sind, jeweils einen Temperaturwert zu messen oder messen zu lassen, und zu jedem der Messintervalle aus den während des Messintervalls gemessenen Temperaturwerten jeweils einen prominenten, z. B. den maximalen, den minimalen, den durchschnittlichen, usw. Temperaturwert zu ermitteln bzw. zu erheben und um die so erhobenen prominenten Temperaturwerte, oder deren Beträge, in die/der Speichervorrichtung zu speichern. Die Steuervorrichtung ist weiter programmiert, um die gespeicherten Temperaturwerte oder deren Beträge aus der Speichervorrichtung auszulesen und ferner, um einen voreingestellten oder vorbestimmten Betrag der Temperatur (also z. B. dessen Höhe, wie sie z. B. auf einer Anzeige der Temperaturmessvorrichtung angezeigt sein könnte), z. B. ebenfalls aus der Speichervorrichtung, auszulesen. Der vorbestimmte Betrag der Temperatur gibt an, welchen Betrag die Temperatur der Behandlungsflüssigkeit an einer vorbestimmten Stelle innerhalb der Fluidleitung stromab der Heizvorrichtung, welche in oder an dieser Fluidleitung angeordnet ist, laut Voreinstellung als Maximalwert betragen darf oder als Sollwert wenigstens betragen muss.

Die Steuervorrichtung ist weiter programmiert, um eine Abkühlung der Behandlungsflüssigkeit bei ihrer Passage zur vorbestimmten Stelle, welche z. B. zwischen dem Temperatursensor und der vorbestimmten Stelle erfolgt, zumindest näherungsweise, zu bestimmen oder anzunehmen.

Die Steuervorrichtung der Blutbehandlungsvorrichtung ist ferner programmiert, um zu ermitteln, z. B. zu berechnen, wie hoch die Förderrate der Pumpe, welche in oder an der Fluidleitung angeordnet ist, maximal sein darf, damit die sich bereits stromab der Heizvorrichtung in der Fluidleitung befindende Behandlungsflüssigkeit, deren erhobene Temperaturwerte, z. B. aus der Speichervorrichtung, ausgelesen wurden, mit einer Temperatur kleiner oder gleich des Maximalwerts für diese Temperatur bzw. für dessen Betrag die vorbestimmte Stelle erreicht. Alternativ oder ergänzend kann die Steuervorrichtung programmiert sein, die Förderrate dieser Pumpe dergestalt zu ermitteln, dass die Temperatur des zu fördernden Fluids an der vorbestimmten Stelle nur unwesentlich von einem Sollwert abweicht, insbesondere größer oder gleich diesem ist, oder innerhalb eines Sollbereichs liegt.

Weiter eine Steuervorrichtung vorgeschlagen, welche wie hierin beschrieben oder offenbart ausgestaltet oder programmiert ist.

Ein digitales, insbesondere nichtflüchtiges, Speichermedium, insbesondere in Form eines maschinenlesbaren Trägers, insbesondere in Form einer Diskette, Speicherkarte, CD, DVD, EPROM, FRAM (Ferroelectric RAM) oder SSD (Solid-State-Drive), insbesondere mit elektronisch oder optisch auslesbaren Steuersignalen, kann derart mit einem programmierbaren Computersystem zusammenwirken, dass eine Blutbehandlungsvorrichtung, z. B. eine herkömmliche Blutbehandlungsvorrichtung, eine nicht-erfindungsgemäße Blutbehandlungsvorrichtung oder eine Blutbehandlungsvorrichtung des Standes der Technik, zu einer erfindungsgemäßen Blutbehandlungsvorrichtung programmiert oder umprogrammiert wird, und/oder dass eine Steuervorrichtung, z. B. eine herkömmliche Steuervorrichtung, eine nicht-erfindungsgemäße Steuervorrichtung oder eine Steuervorrichtung des Standes der Technik, zu einer erfindungsgemäßen Steuervorrichtung programmiert oder umprogrammiert wird.

Ein Computerprogramm-Produkt weist einen volatilen, flüchtigen oder auf einem maschinenlesbaren Träger gespeicherten Programmcode oder eine Signalwelle auf, welcher derart mit einem programmierbaren Computersystem zusammenwirken kann, dass eine Blutbehandlungsvorrichtung, z. B. eine herkömmliche Blutbehandlungsvorrichtung, eine nicht-erfindungsgemäße Blutbehandlungsvorrichtung oder eine Blutbehandlungsvorrichtung des Standes der Technik, zu einer erfindungsgemäßen Blutbehandlungsvorrichtung programmiert oder umprogrammiert wird, und/oder dass eine Steuervorrichtung, z. B. eine herkömmliche Steuervorrichtung, eine nicht-erfindungsgemäße Steuervorrichtung oder eine Steuervorrichtung des Standes der Technik, zu einer erfindungsgemäßen Steuervorrichtung programmiert oder umprogrammiert wird.

Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Server-system, Cloud Computing System, etc.) oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen, sowie jeder andere hierin genannte Speicher oder jedes andere hierin genannte Speichermedium sein.

Ein erfindungsgemäßes Computerprogramm umfasst einen Programmcode, mittels welchem veranlasst wird, dass eine Blutbehandlungsvorrichtung, z. B. eine herkömmliche Blutbehandlungsvorrichtung, eine nicht-erfindungsgemäße Blutbehandlungsvorrichtung oder eine Blutbehandlungsvorrichtung des Standes der Technik, zu einer erfindungsgemäßen Blutbehandlungsvorrichtung programmiert oder umprogrammiert wird, und/oder dass eine Steuervorrichtung, z. B. eine herkömmliche Steuervorrichtung, eine nicht-erfindungsgemäße Steuervorrichtung oder eine Steuervorrichtung des Standes der Technik, zu einer erfindungsgemäßen Steuervorrichtung programmiert oder umprogrammiert wird.

Erfindungsgemäße Ausführungsformen können manche, einige oder alle der folgenden Merkmale in beliebiger Kombination aufweisen, soweit dies für den Fachmann nicht erkennbar technisch unmöglich ist.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin von Raumangaben, wie z. B. von "oben", "unten", "links" oder "rechts", die Rede ist, versteht der Fachmann hierunter die Anordnung in den hier angehängten Figuren und/oder im Gebrauchszustand. "Unten" ist dem Erdmittelpunkt oder dem unteren Rand der Figur näher als "oben".

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Wenn hierin Verfahrensschritte genannt sind, so ist die erfindungsgemäße Steuervorrichtung in einigen Ausführungsform konfiguriert, um einen, mehrere oder alle dieser Verfahrensschritte automatisch zu veranlassen oder ausführen zu lassen.

Wenn hierin Verfahrensschritte genannt sind, so weist die erfindungsgemäße Steuervorrichtung oder die erfindungsgemäße Blutbehandlungsvorrichtung entsprechende Vorrichtungen auf, welche sich vorzugsweise namentlich an die Bezeichnung des jeweiligen Verfahrensschritts anlehnen (z. B. "Ermitteln" als Verfahrensschritt und "Vorrichtung zum Ermitteln" für die Vorrichtung, usw.) und welche ebenfalls Teil der erfindungsgemäßen Vorrichtungen sein oder hiermit in Signalverbindung verbunden sein können, um sie entsprechend anzusteuern.

Wenn hierin von programmiert oder konfiguriert die Rede ist, so können diese Begriffe in manchen Ausführungsformen gegeneinander austauschbar sein.

Wenn hierin von einer Signal- oder Kommunikationsverbindung zweier Komponenten die Rede ist, so kann hierunter eine im Gebrauch bestehende Verbindung zu verstehen sein. Ebenso kann hierunter zu verstehen sein, dass eine Vorbereitung zu einer solchen (kabelgebundenen, kabellosen oder auf andere Weise umgesetzten) Signalverbindung besteht, beispielsweise durch eine Kopplung beider Komponenten, etwa mittels *pairing,* usw.

Unter *pairing* versteht man einen Prozess, der im Zusammenhang mit Rechnernetzwerken erfolgt, um eine anfängliche Verknüpfung zwischen Rechnereinheiten zum Zwecke der Kommunikation herzustellen. Das bekannteste Beispiel hierfür ist das Herstellen einer Bluetooth-Verbindung, mittels welcher verschiedene Einrichtungen (z. B. Smartphone, Kopfhörer) miteinander verbunden werden. *Pairing* wird gelegentlich auch als *bonding* bezeichnet.

Die Steuervorrichtung kann die Ausführung aller oder im Wesentlichen aller Verfahrensschritte veranlassen. Ein hierin beschriebenes Verfahren kann im Wesentlichen oder vollständig von der Steuervorrichtung ausgeführt werden. Es kann teilweise von der Steuervorrichtung ausgeführt werden, insbesondere können jene Schritte von der Steuervorrichtung ausgeführt werden, welche ein menschliches Zutun und/oder ein Bereitstellen nicht erfordern oder betreffen. Die Steuervorrichtung kann als reine Steuervorrichtung oder auch als Regelvorrichtung dienen.

In einigen Ausführungsformen liegt die Steuervorrichtung in oder an der Blutbehandlungsvorrichtung vor, etwa gemeinsam mit weiteren Komponenten oder Vorrichtungen der Blutbehandlungsvorrichtung in einem gemeinsamen Gehäuse der Blutbehandlungsvorrichtung.

In manchen Ausführungsformen der erfindungsgemäßen Blutbehandlungsvorrichtung ist die Pumpe zum Fördern der Behandlungsflüssigkeit eine Dialysierflüssigkeitspumpe, welche in oder an einer Dialysierflüssigkeitszulaufleitung angeordnet ist, um Dialysierflüssigkeit als Behandlungsflüssigkeit in eine Dialysierflüssigkeitskammer eines Blutfilters oder Dialysators zu fördern, wobei der Blutfilter neben der Dialysierflüssigkeitskammer eine, von dieser mittels einer Membran getrennte, Blutkammer aufweist.

In einigen Ausführungsformen ist die Pumpe eine Substituatpumpe, welche in oder an einer Substituatleitung angeordnet ist, um Substituat als Behandlungsflüssigkeit dem extrakorporalen Blutkreislauf der Blutbehandlungsvorrichtung (entweder in Prä- oder in Postdilution) an hierfür vorgesehenen Zugabestellen zuzuführen.

In manchen Ausführungsformen ist die vorbestimmte Stelle die Dialysierflüssigkeitskammer des Blutfilters oder ein Einlass in den Blutfilter bzw. die Dialysierflüssigkeitskammer.

In einigen Ausführungsformen ist die vorbestimmte Stelle eine Zugabestelle für Behandlungsflüssigkeit als Substituat in den extrakorporalen Blutkreislauf.

In manchen Ausführungsformen ist die Länge bzw. Dauer der Messintervalle konstant, beispielsweise jeweils 15 Sekunden. Die Länge bzw. Dauer kann in der Speichervorrichtung gespeichert sein.

In einigen Ausführungsformen werden die Beträge der in den Messintervallen jeweils in der Fluidleitung geförderten Mengen an Behandlungsflüssigkeit, beispielsweise in der Speichervorrichtung, gespeichert.

In manchen Ausführungsformen werden die Beträge der in den Messintervallen jeweils in der Fluidleitung geförderten Mengen an Behandlungsflüssigkeit gemeinsam mit dem erhobenen prominenten Temperaturwert und einer Bezeichnung zum Identifizieren des zugehörigen Messintervalls, für welches der Temperaturwert jeweils erhoben wurde, gespeichert.

In einigen Ausführungsformen wird die Abkühlung der Behandlungsflüssigkeit bei ihrer Passage zwischen der Temperaturmessvorrichtung bzw. ihrem Temperatursensor und der vorbestimmten Stelle anhand von einer oder mehreren Tabellen, vorzugsweise Nachschlagetabellen, durch Berechnung(en) ermittelt oder zumindest näherungsweise bestimmt oder angenommen.

In manchen Ausführungsformen ist die Speichervorrichtung ein Historybuffer, ein Ringpuffer, ein Ringspeicher oder dergleichen, oder weist einen solchen auf, in welchem die erhobenen Temperaturwerte, gespeichert oder zu speichern sind.

Die Speichervorrichtung kann mit z. B. 30 Elementen ausgelegt sein. Jedes Element beinhaltet optional für ein Messintervall (von z. B. 15 Sekunden), den in diesem Zeitraum mittels der Temperaturmessvorrichtung bzw. ihres Temperatursensors gemessenen, maximalen oder anderweitig prominenten Temperaturwert sowie das in diesem Intervall geförderte Behandlungsflüssigkeitsvolumen, sofern letzteres nicht z. B. konstant gehalten wurde.

In einigen Ausführungsformen ist die Steuervorrichtung programmiert, um die Förderrate der Pumpe zum Fördern von Behandlungsflüssigkeit derart zu begrenzen oder anderweitig zu steuern oder regeln, dass die Temperatur, welche die von ihr geförderte Behandlungsflüssigkeit, wenn sie an die vorbestimmte Stelle gefördert ist, aufweist, einen vorbestimmten, für den Patienten vorab als unbedenklich eingeschätzten Temperaturwert, z. B. 41°C, an dieser vorbestimmten Stelle nicht überschreitet.

In manchen Ausführungsformen ist die Steuervorrichtung programmiert, um die erhobenen Temperaturwerte bzw. ihre Beträge, insbesondere nach ihrer Speicherung, für Zeiträume, in welchen die Pumpe zum Fördern von Behandlungsflüssigkeit angehalten ist und damit nicht fördert, mit einem Korrekturfaktor nach unten zu korrigieren.

Auf diese Weise wird bei gestoppter Pumpe z. B. ein virtueller Mindestfluss angenommen, um eine Abkühlung der Behandlungsflüssigkeit in der Fluidleitung zu simulieren.

In einigen Ausführungsformen ist die Blutbehandlungsvorrichtung als Dialysevorrichtung, Hämodialysevorrichtung, Hämofiltrationsvorrichtung oder Hämodiafiltrationsvorrichtung, insbesondere als eine Vorrichtung für die akute, die chronische Nierenersatztherapie oder für die kontinuierliche Nierenersatztherapie (CRRT = continuous renal replacement therapy) ausgestaltet.

In manchen Ausführungsformen ist die Blutbehandlungsvorrichtung verbunden oder aufgerüstet mit einem Blutschlauchsatz und/oder einer Fluidleitung für die Behandlungsflüssigkeit, welcher/welche für die pädiatrische Blutbehandlung vorgesehen oder zugelassen ist. In anderen Ausführungsformen ist die Blutbehandlungsvorrichtung verbunden oder aufgerüstet mit einem Blutschlauchsatz und/oder einer Fluidleitung für die Behandlungsflüssigkeit, welcher/welche für die adulte Blutbehandlung vorgesehen oder zugelassen ist.

In einigen Ausführungsformen ist ein Messintervall ein Zeitintervall, vorzugsweise vorbestimmter Länge, welche die Länge vorzugsweise auch der übrigen betrachteten Zeitintervalle hat, innerhalb welchem die Temperatur in einer Vielzahl von Messungen wiederholend ermittelt wird, um für dieses Zeitintervall den hierin offenbarten prominenten Temperaturwert zu erheben.

In manchen Ausführungsformen kann vorgesehen sein, einen übergeordneten prominenten Temperaturwert für die Behandlungsflüssigkeit für das vollständige Volumen (das der Gesamtheit der Teilvolumina entspricht) zwischen Temperatursensor und vorbestimmter Stelle zu ermitteln, mit anderen Worten einen prominenten Temperaturwert jenes Volumens, welches den Temperatursensor bereits, die vorbestimmte Stelle aber noch nicht passiert hat. Das vollständige Volumen könnte sich beispielsweise aus dem Volumen eines Heizbeutels stromab der Temperaturmessvorrichtung oder ihres Temperatursensors und dem Volumen der sich an den Heizbeutel anschließenden Fluidleitung, z. B. bis zur vorbestimmten Stelle, zusammensetzen. So würde sich beispielsweise bei einem Volumen innerhalb des Heizbeutels aber stromab der Temperaturmessvorrichtung von 30 ml und einem Volumen der sich an den Heizbeutel anschließenden Fluidleitung von 5 ml demnach ein vollständiges Volumen von 35 ml ergeben. Um den übergeordneten prominenten Temperaturwert der Behandlungsflüssigkeit bzw. dessen Betrag, also bezogen auf das vollständige oder gesamte Volumen oder Lumen zu ermitteln, kann beispielsweise aus den für jedes Messintervall erhobenen prominenten Temperaturwerten weiter ein übergeordneter prominenter, z. B. maximaler, Temperaturwert für das vollständige Volumen erhoben bzw. ermittelt werden. Die Prominenz dieses Temperaturwerts kann, muss aber nicht, die gleiche Prominenz sein wie bei der Erhebung der prominenten Temperaturwerte der einzelnen Messintervalle. Anders ausgedrückt, ist beispielsweise bei den einzelnen Messintervallen jeweils ein maximaler Temperaturwert aus den gemessenen Temperaturen als prominenter Temperaturwert erhoben worden, so kann analog hierzu wiederum ein maximaler Temperaturwert als übergeordneter prominenter Temperaturwert aus diesen prominenten Temperaturwerten für das vollständige Volumen zwischen Temperatursensor und vorbestimmter Stelle erhoben werden. Dies ist aber nicht beschränkend zu verstehen; es könnte beispielsweise alternativ ein Durchschnitt oder dergleichen als übergeordneter prominenter Temperaturwert bestimmt werden, der seinerseits jedoch auch aus einer Vielzahl von maximalen Temperaturwerten ermittelt wurde, usw., oder umgekehrt.

Manche oder alle erfindungsgemäßen Ausführungsformen können einen, mehrere oder alle der oben und/oder im Folgenden genannten Vorteile aufweisen.

Ist, wie vorgesehen sein kann, die Heizleistung der Heizvorrichtung einer temperaturbasierten Regelung oder Begrenzung unterworfen, so kann sichergestellt sein, dass die Temperatur an der vorbestimmten Stelle eine maximale Temperatur nicht übersteigt und/oder eine Solltemperatur nicht unterschreitet. Mittels einer entsprechenden Anpassung der Förderrate der Pumpe zum Fördern der Behandlungsflüssigkeit mit Blick auf vorstehende Temperaturwerte kann somit der Patient vor den Folgen des Gebrauchs einer Behandlungsflüssigkeit von ungeeigneter Temperatur geschützt werden.

In manchen Ausführungsformen ist die Bestimmung dieses maximal erlaubten Flüssigkeitsflusses, z. B. Dialysat- bzw. Substituatflusses, und die Kenntnis der Temperatur der Flüssigkeit im Bereich zwischen der Heizvorrichtung und der vorbestimmten Stelle möglich, ohne dass es hierfür eines weiteren Sensors bedarf.

Wird bei einer Dialysebehandlung die Behandlungsflüssigkeit erwärmt, muss die Temperatur der Flüssigkeiten an vorbestimmten Stellen, insbesondere an der Zugabestelle zum Blutsystem (beispielsweise am Einlass des Dialysators bzw. einer Zugabestelle für Substituat) den Vorgaben des Herstellers entsprechen. Dabei kann erfindungsgemäß genügen, die Temperatur nur in der Heizvorrichtung zu messen, wo üblicherweise bereits eine Temperaturmessvorrichtung bzw. ein Temperatursensor vorgesehen ist, und nicht auch noch an der vorbestimmten Stelle, wo üblicherweise keine weitere Temperaturmessvorrichtung vorhanden ist.

Dennoch ist die Temperatur bzw. ein Wärmeverlust der Behandlungsflüssigkeit an oder bis zum Erreichen der vorbestimmten Stelle bekannt, und es können die Flüsse der Behandlungsflüssigkeit entsprechend geregelt werden.

Die in den Heizvorrichtungen beheizte Behandlungsflüssigkeit kann sich dabei bis zur entsprechenden vorbestimmten Stelle merklich abkühlen. Der Wärmeverlust ist hierbei im Wesentlichen von vier Faktoren abhängig:
- Umgebungstemperatur
- Luftzirkulation
- Temperatur der zu beheizenden Behandlungsflüssigkeit im Beutel
- eingestellter Behandlungsflüssigkeitsfluss

Um diesem Wärmeverlust, also einer Abkühlung, auf dem Weg zur vorbestimmten Stelle entgegenzuwirken, wird in manchen Ausführungsformen vorteilhaft die Solltemperatur (und somit die Solltemperatur für den Regelalgorithmus der Heizung) entsprechend angepasst, sprich erhöht. Dies ist möglich in Kenntnis der Wärmeverluste und mit Blick darauf, dass eine - nachträglich gesehen - möglichweise doch zu hoch gewählte Temperatur am Heizbeutelausgang über die hierin beschriebene Regelung der Flussgeschwindigkeit innerhalb der Fluidleitung mittels der Regelung der Förderrate der fördernden Fluidpumpe für den Patienten letztlich unschädlich ist, da insbesondere die Flussgeschwindigkeit in der Fluidleitung einen starken Einfluss darauf hat, wie stark die Behandlungsflüssigkeit auf dem Weg zur vorbestimmten Stelle abkühlt. Beispielsweise muss bei geringen Flussgeschwindigkeiten die Temperatur der Behandlungsflüssigkeit am Ausgang der Heizvorrichtung deutlich höher sein als bei höheren Flussgeschwindigkeiten, u. a. wegen entsprechend längerer Verweilzeit in der Fluidleitung bei geringeren Flussgeschwindigkeiten.

Im Umkehrschluss würde eine plötzliche Erhöhung der Flussgeschwindigkeit dazu führen, dass die Temperatur an der vorbestimmten Stelle deutlich höher als die gewünschte Temperatur ist und ggf. sogar zu einer Patientengefährdung führen kann. Mittels der vorliegenden Erfindung kann aufgrund der maximalen Temperatur im Bereich nach dem Temperatursensor vorteilhaft der zulässige Fluss berechnet oder z. B. näherungsweise bestimmt werden, etwa durch Nachschlagen in einer Nachschlagetabelle, bei dem sich über die Abkühlung der Behandlungsflüssigkeit innerhalb der Fluidleitung noch ein zulässiger Temperaturwert derselben an der vorbestimmten Stelle ergibt. Dadurch kann hier mit dem maximal möglichen Fluss weiter behandelt werden und somit vorteilhaft eine möglichst große Behandlungseffektivität erreicht werden. Ein Drosseln der Heizvorrichtung bzw. ein Reduzieren des Behandlungsflüssigkeitsflusses, wie es bisher notwendig war, um einen gewünschten Temperaturwert für die Behandlungsflüssigkeit an der vorbestimmten Stelle zu erreichen, und was zu einer herabgesetzten Effektivität der Behandlung führte, kann so weitgehend umgangen oder zumindest vorteilhaft gesteuert werden.

Im Folgenden wird die vorliegende Erfindung unter Bezugnahme auf die beigefügten Figuren rein exemplarisch beschrieben. In ihnen bezeichnen gleiche Bezugszeichen gleiche oder ähnliche Komponenten. Es gilt:
- **Fig. 1**: zeigt schematisch sehr vereinfacht ein Flussbild einer kontinuierlichen veno-venösen Hämodiafiltration (Continuous Veno-Venous Hemodiafiltration, CVVHDF) mit Postdilution einer erfindungsgemäßen Blutbehandlungsvorrichtung in einer beispielhaften Ausführungsform;
- **Fig. 2**: zeigt Komponenten einer erfindungsgemäßen Blutbehandlungsvorrichtung in einer beispielhaften Ausführungsform; und
- **Fig. 3**: zeigt schematisch vereinfacht einen Fluidleitungsaufbau einer erfindungsgemäßen Blutbehandlungsvorrichtung in einer weiteren beispielhaften Ausführungsform.

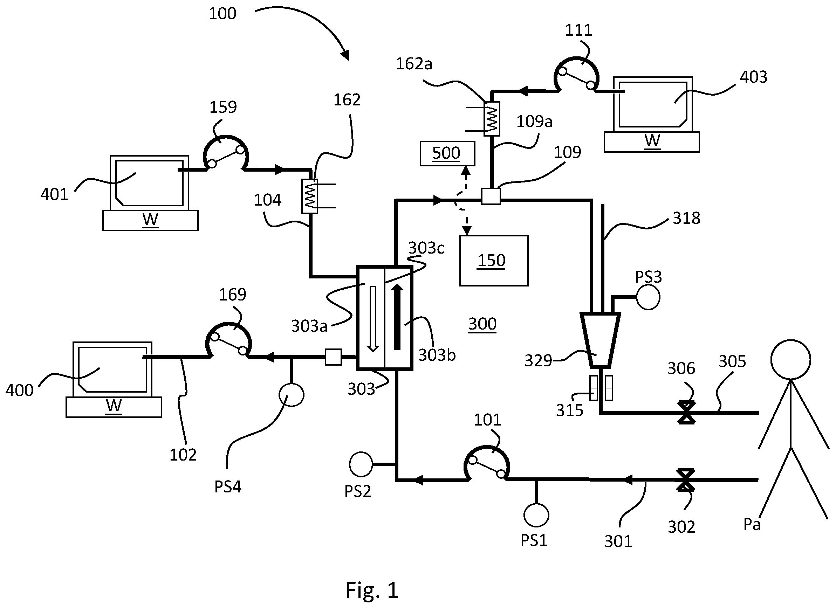

**Fig. 1** zeigt schematisch sehr vereinfacht ein Flussbild einer kontinuierlichen veno-venösen Hämodiafiltration (Continuous Veno-Venous Hemodiafiltration, CVVHDF) mit Postdilution einer erfindungsgemäßen Blutbehandlungsvorrichtung 100 in einer ersten beispielhaften Ausführungsform.

Die erfindungsgemäße Blutbehandlungsvorrichtung 100 (in Fig. 1 nur durch einzelne, schematisch stark vereinfachte Komponenten dargestellt) weist eine Blutpumpe 101 zum Fördern von Blut entlang eines Blutschlauchsatzes oder eines extrakorporalen Blutkreislaufs 300, durch diesen hindurch oder innerhalb desselben, auf, welcher seinerseits optional einen venösen Blasenfänger 329 aufweist.

Der optionale venöse Blasenfänger 329 kann eine Entlüftungseinrichtung 318 aufweisen und mit dem Drucksensor PS3 in Fluidverbindung stehen. Die Anordnung der Fig. 1 umfasst einen optionalen Luftblasendetektor 315 zum Detektieren von Luft und/oder Blut. Er ist hier stromab des venösen Blasenfängers 329 angeordnet.

Der Blutschlauchsatz 300 steht mit seinem arteriellen Leitungsabschnitt 301 (auch als erste Leitung, arterielle Patientenleitung oder Blutentnahmeleitung bezeichnet) mit einer arteriellen Patientenschlauchklemme 302 der Blutbehandlungsvorrichtung 100 in Wechselwirkung und kann durch Letztere verschlossen werden. Er weist ferner Verbinder für eine arterielle Konnektionsnadel (in Fig. 1 nicht dargestellt) oder einen Anschluss für einen zentralvenösen Katheter auf oder ist hiermit verbunden.

Weiter kann der Blutschlauchsatz 300 an seinem venösen Leitungsabschnitt 305 (auch als venöse Patientenleitung, Blutrückgabeleitung oder zweite Leitung bezeichnet) mittels einer venösen Patientenschlauchklemme 306 der Blutbehandlungsvorrichtung 100 verschlossen werden, und weist Verbinder für eine venöse Konnektionsnadel (in Fig. 1 nicht dargestellt) oder einen Anschluss für einen zentralvenösen Katheter auf oder ist hiermit verbunden. Die optionalen Konnektionsnadeln sind zu ihrer Verbindung mit dem Blutkreislauf eines Patienten Pa vorgesehen.

Im Beispiel der Fig. 1 ist eine Ausführungsform einer erfindungsgemäßen Steuervorrichtung 150 angedeutet, welche konfiguriert ist, die Blutbehandlungsvorrichtung 100 zu steuern oder zu regeln, insbesondere deren Blutpumpe 101, eine erste Flusspumpe 159 und/oder eine Substituatpumpe 111.

Der extrakorporale Blutkreislauf oder Blutschlauchsatz 300 weist im Beispiel der Fig. 1 weiter einen Blutfilter 303 oder Dialysator auf, dessen Blutkammer 303b mit dem arteriellen Leitungsabschnitt 301 und mit dem venösen Leitungsabschnitt 305 verbunden ist. Eine Dialysierflüssigkeitskammer 303a des Blutfilters 303 ist mit der zur Dialysierflüssigkeitskammer 303a führenden Dialysierflüssigkeitszulaufleitung 104 und einer von der Dialysierflüssigkeitskammer 303a wegführenden Dialysatablaufleitung 102, welche Dialysat, also verbrauchte Dialysierflüssigkeit, leitet, verbunden. Hierzu dienen geeignete Konnektoren an der Dialysierflüssigkeitszulaufleitung 104 bzw. an der Dialysatablaufleitung 102 einerseits und an den Dialysatports andererseits, welche, insbesondere lösbar, miteinander verbunden werden können.

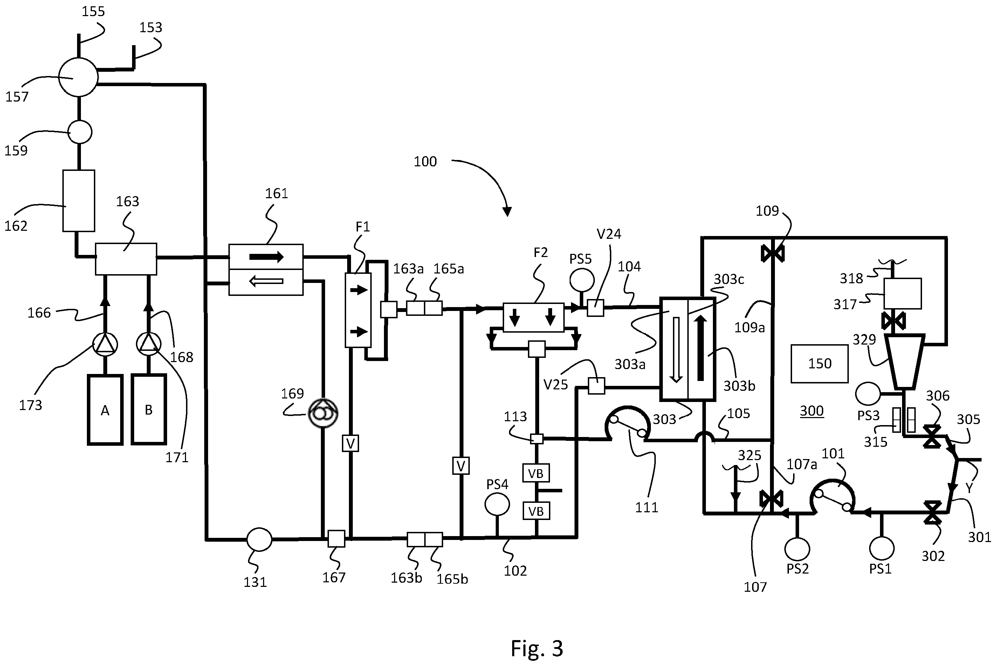

Dialysierflüssigkeitskammer 303a und Blutkammer 303b sind durch eine zumeist semi-permeable Membran 303c voneinander getrennt. Blut und Dialysierflüssigkeit werden zumeist im Gegenstromprinzip durch den Blutfilter 303 geleitet. Das Blut wird im Blutfilter 303 gereinigt. Die semi-permeable Membran stellt die Trennscheide zwischen der Blutseite mit dem extrakorporalen Blutkreislauf 300 und der Maschinenseite mit dem Dialysierflüssigkeits- bzw. Dialysatkreislauf dar, die in Fig. 1 und Fig. 3 links der Membran 303c dargestellt ist. Zu Fig. 3 wird die Maschinenseite in einer weiteren Ausführungsform genauer beschrieben.

Mittels der ersten Flusspumpe 159 wird im Beispiel der Fig. 1 über die Dialysierflüssigkeitszulaufleitung 104 (als Beispiel einer Fluidleitung) frische Dialysierflüssigkeit (als Beispiel einer Behandlungsflüssigkeit) aus einer Dialysierflüssigkeitsquelle 401, hier einem Beutel mit Dialysierflüssigkeit, zur Dialysierflüssigkeitskammer 303a gefördert. Die Dialysierflüssigkeit kann mittels einer Heizvorrichtung 162 angewärmt oder auf eine vorbestimmte Temperatur gebracht werden.

Das Dialysat, welches auch als Effluent bezeichnet wird und verbrauchte Dialysierflüssigkeit, ggf. angereichert mit Filtrat, ist oder umfasst, verlässt die Dialysierflüssigkeitskammer 303a des Blutfilters 303 über die Dialysatablaufleitung 102, optional gefördert mittels einer zweiten Flusspumpe 169.

Im Beispiel der Fig. 1 wird das Effluent zunächst in einem optionalen Effluentbeutel 400 aufgefangen, um anschließend in ein Waschbecken oder einen anderen geeigneten Ausguss hinein verworfen zu werden.

Mittels Waagen W können jeweils die zugeführte Menge an Dialysierflüssigkeit bzw. die abgeführte Menge an Effluent (auch: Filtrat) ermittelt werden. Die Waagen W, bzw. ihre Messwerte, dienen der Bilanzierung.

Mittels eines Postdilutionsventils 109 wird dem extrakorporalen Blutkreislauf 300 Substituat (als weiteres Beispiel einer Behandlungsflüssigkeit) aus einer Substituatquelle 403, hier einem Substituatbeutel, zugeführt. Dies wird von der Substituatpumpe 111, die in oder an der zum Postdilutionsventil gehörenden Leitung 109a (als weiteres Beispiel einer Fluidleitung) angeordnet ist, gefördert, optional wird das Substituat in einer Heizvorrichtung 162a angewärmt oder auf eine vorbestimmte Temperatur gebracht. Mittels der Waage W kann die zugeführte Menge an Substituat ermittelt werden. Die von der Waage W gelieferten Werte können ebenfalls der Bilanzierung dienen.

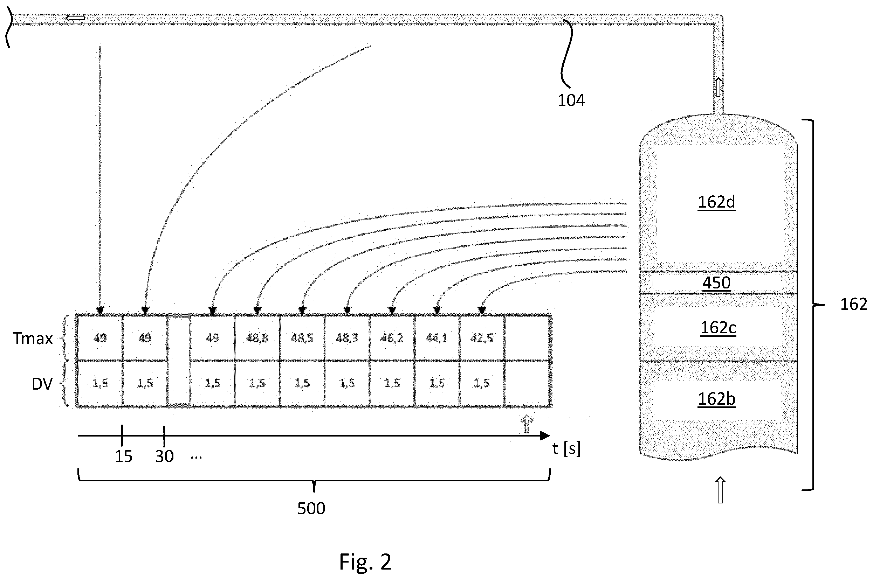

Mittels der erfindungsgemäßen Steuervorrichtung 150 kann eine Tempermessvorrichtung 450 (hier nicht gezeigt, siehe vielmehr Fig. 2) an jeder beliebigen der Heizvorrichtungen 162, 162a veranlasst werden, jeweils für ein Messintervall z. B. einen maximalen Temperaturwert Tmax als prominenten Temperaturwert zu erheben und in einer Speichervorrichtung 500 zu speichern. Dies wird detaillierter zu Fig. 2 beschrieben.

Die Steuervorrichtung 150 kann weiter programmiert bzw. konfiguriert sein, um die gespeicherten Temperaturwerte aus der Speichervorrichtung 500 auszulesen.

Weiter kann die Steuervorrichtung 150 konfiguriert sein, einen voreingestellten oder vorbestimmten Betrag der Temperatur, welche die Dialysierflüssigkeit an einer vorbestimmten Stelle innerhalb der Dialysierflüssigkeitszulaufleitung 104 stromab der Heizvorrichtung 162, beispielsweise am Einlass der Dialysierflüssigkeitszulaufleitung 104 in die Dialysierflüssigkeitskammer 303a des Dialysators 303, laut Voreinstellung als Maximalwert höchstens betragen darf, aus der Speichervorrichtung 500 auszulesen.

Alternativ oder ergänzend kann die Steuervorrichtung 150 konfiguriert sein, einen voreingestellten oder vorbestimmten Betrag der Temperatur, welche das Substituat an einer vorbestimmten Stelle innerhalb der Substituatleitung 109a stromab der Heizvorrichtung 162a, beispielsweise der Zugabestelle 109 für Substituat in den extrakorporalen Blutschlauchsatz 300, laut Voreinstellung als Maximalwert betragen darf, aus der Speichervorrichtung 500 auszulesen.

Dies gilt analog für Sollwerte, die die Temperaturwerte der Behandlungsflüssigkeit jeweils an der vorbestimmten Stelle mindestens aufweisen sollen.

Ein Abschätzen einer Abkühlung der Dialysierflüssigkeit bei ihrer Passage zwischen der Temperaturmessvorrichtung 450 bzw. ihrem Temperatursensor und der vorbestimmten Stelle und ein Ermitteln, wie hoch die Förderrate der Pumpe 159, 111 jeweils maximal sein darf, damit die sich bereits stromab der Heizvorrichtung 162 in der Dialysierflüssigkeitszulaufleitung 104 befindende Dialysierflüssigkeit bzw. das sich bereits stromab der Heizvorrichtung 162a in der Substituatleitung 109a befindende Substituat die jeweils vorbestimmte Stelle mit einer maximal zulässigen Temperatur erreicht, wird zu Fig. 2 näher beschrieben.

Weitere Druckmesseinrichtungen PS1, PS2, PS4 können gemeinsam oder unabhängig voneinander optional an den verschiedenen Leitungen angeordnet sein.

Fig. 2 zeigt rechts die Heizvorrichtung 162 einer erfindungsgemäßen Blutbehandlungsvorrichtung 100 in einer beispielhaften Ausführungsform. Die Heizvorrichtung 162 dient im Beispiel der Fig. 2 zum Erwärmen der Dialysierflüssigkeit als Beispiel einer Behandlungsflüssigkeit innerhalb einer Fluidleitung, hier der Dialysierflüssigkeitszulaufleitung 104. Die Behandlungsflüssigkeit der Fig. 2 wird von einer ersten Flusspumpe 159 (in der Figur nicht gezeigt, siehe Fig. 1) durch die Heizvorrichtung 162 und die Fluidleitung gefördert.

Die Heizvorrichtung 162 ist optional als Heizbeutel dargestellt, welcher einen beheizten Bereich 162b, optional einen nicht beheizten Bereich 162c, eine Temperaturmessvorrichtung 450, hier exemplarisch in Form eines Ringsensors, sowie ein Volumen 162d stromabwärts des Ringsensors, beispielsweise von ca. 30 ml, aufweist.

Die Temperaturmessvorrichtung 450 dient zum Erheben, z. B. Messen oder Ermitteln, der Temperatur der Dialysierflüssigkeit, wenn sie in der Heizvorrichtung 162 vorliegt oder an der Temperaturmessvorrichtung 450 bzw. an deren Temperatursensor vorbeiströmt.

Mittels der erfindungsgemäßen Steuervorrichtung 150 (in Fig. 2 nicht gezeigt, siehe hierzu Fig. 1) wird die Temperaturmessvorrichtung 450 veranlasst, zu einer Vielzahl von Messzeitpunkten, hier dargestellt entlang der Zeitachse t jeweils innerhalb eines bestimmten Messintervalls (oder Zeitintervalls), hier beispielsweise 15 Sekunden, einen maximalen Temperaturwert Tmax zu erheben und in der Speichervorrichtung 500 zu speichern. Diese ist mit beispielhaften Temperaturwerten Tmax links neben der Heizvorrichtung 162 dargestellt. Die Speicherung der Temperaturwerte Tmax bzw. deren Beträge kann hierin auch als Historybuffer bezeichnet sein.

Die Steuervorrichtung 150 kann weiter programmiert bzw. konfiguriert sein, um die gespeicherten Temperaturwerte aus der Speichervorrichtung 500 auszulesen.

Weiter kann in der Steuervorrichtung 150 oder der Speichervorrichtung 500 ein voreingestellter oder vorbestimmter Betrag der Temperatur, welche die Behandlungsflüssigkeit, hier Dialysierflüssigkeit, an einer vorbestimmten Stelle innerhalb der Fluidleitung, hier die Dialysierflüssigkeitszulaufleitung 104, stromab der Heizvorrichtung 162 laut Voreinstellung als Maximalwert betragen oder nicht übersteigen darf, hinterlegt sein, um mittels der Steuervorrichtung 150 aus der Speichervorrichtung 500 ausgelesen zu werden. Analoges gilt ebenfalls für Sollwerte der Temperatur, insbesondere Mindesttemperatur, oder einen Sollbereich für die Temperatur an der vorbestimmten Stelle.

Die Speichervorrichtung 500 kann beispielsweise zum Speichern einer Temperaturhistorie vorgesehen sein. Die Temperaturhistorie kann wiederum eine vorgegebene Anzahl von erhobenen, prominenten Temperaturwerten, im Folgenden rein exemplarisch als im Messintervall jeweils erhobene maximale Temperaturwerte Tmax bezeichnet, sein oder umfassen. Anstelle von Maximaltemperaturwerten könnten auch die mittleren, durchschnittlichen, niedrigsten oder andere vorbestimmte, prominente Temperaturwerte erhoben und gespeichert werden.

In manchen Ausführungsformen ist die Speichervorrichtung 500 zum Speichern von beispielsweise 30 prominenten Temperaturwerten Tmax programmiert, wobei jeder gespeicherte Temperaturwert Tmax beispielsweise die maximale, in einem fest vorgegebenen Messintervall, beispielsweise 15 Sekunden, gemessene Temperatur ist, und wobei diese Messintervalle sich jeweils aneinander anschließen, also aufeinander folgend sein können.

Aufgrund des vorgegebenen Messintervalls wird der Temperaturwert Tmax indirekt einem bestimmten Teilvolumen DV der Dialysierflüssigkeit entlang des Wegs der Flüssigkeit durch die Fluidleitung zugeordnet, wobei die Modellvorstellung von einer idealen Pfropfenströmung ausgeht, d. h. dass die Flussgeschwindigkeit fast überall im Strömungsquerschnitt der Fluidleitung, hier der Dialysierflüssigkeitszulaufleitung 104, gleich hoch ist. Somit wird im Beispiel der Fig. 2 die Temperaturhistorie aus einem Zeitraum von 30 x 15 s = 450 s gespeichert.

Sobald z. B. die 30 Temperaturwerte Tmax, oder andere promiente Temperaturwerte, in der Speichervorrichtung 500 gespeichert sind, wird beispielsweise im nächsten Messintervall der älteste Temperaturwert Tmax durch einen neuen Temperaturwert überschrieben. Die Speicherplätze der Speichervorrichtung 500 werden in einigen Ausführungsformen somit fortlaufend dergestalt überschrieben, so dass stets nur die jüngsten 30 erhobenen, prominenten Temperaturwerte in chronologischer Reihenfolge ihres Auftretens gespeichert werden.

Zusätzlich kann der in dem Messintervall - messbar z. B. aufgrund der Pumpenumdrehung - geförderte Betrag des Dialysierflüssigkeitsvolumens DV, hierin auch als der Betrag der Menge an Behandlungsflüssigkeit bezeichnet, in der Speichervorrichtung 500 gespeichert werden.

Aufgrund der in der Speichervorrichtung 500 gespeicherten Temperaturwerte Tmax der Temperaturhistorie kann in der Modellvorstellung "vorausschauend" bestimmt werden, wann ein bestimmtes Teilvolumen mit einer bestimmten Temperatur die vorbestimmte Stelle erreicht. Bis zum Erreichen der vorbestimmten Stelle erfolgte Abkühlung entlang des Förderwegs kann hierbei mitberücksichtigt sein.

Die gezeigten Pfeile stellen eine Zuordnung der in der Dialysierflüssigkeitszulaufleitung 104 befindlichen Behandlungsflüssigkeit zu den erhobenen prominenten Temperaturwerten Tmax und dem im Messintervall geförderten Menge DV an Behandlungsflüssigkeit, hier Dialysierflüssigkeit, dar.

In dem hier dargestellten Beispiel für die Messung von Temperaturwerten Tmax mit Hilfe der Temperaturmessvorrichtung 450 sinken die tatsächlichen Temperaturwerte dort im Laufe der Zeit ab, während die in dem Messintervall geförderte Menge DV an Behandlungsflüssigkeit gleich bleibt. Die Erfindung ist allerdings nicht darauf beschränkt. So sind erfindungsgemäß auch Ausführungsformen umfasst, bei denen die im Messintervall geförderte Menge DV an Behandlungsflüssigkeit beispielsweise durch eine erfindungsgemäß ermittelte Förderrate variiert. Ergänzend oder alternativ sind auch Ausführungsformen von der vorliegenden Erfindung umfasst, bei denen die von der Temperaturmessvorrichtung 450 gemessene Temperatur stabil bleibt.

Stoppt die erste Flusspumpe 159 innerhalb eines beispielhaften Messintervalls von beispielsweise 15 Sekunden, wird das Messintervall beim Wiederanlaufen der ersten Flusspumpe 159 beispielsweise fortgesetzt.

Das Innenvolumen einer pädiatrischen Dialysierflüssigkeitszulaufleitung 104 zwischen dem Ausgang des Heizbeutels und dem Einlass der Dialysierflüssigkeitskammer 303a des Dialysators 303 beträgt beispielsweise ca. 5 ml. Bei einer Dialysierflüssigkeitszulaufleitung 104 für die Behandlung eines Erwachsenen beträgt dieses Volumen ca. 10 ml. Diese Größen können in die Berechnung eines Wärmeverlusts einfließen.

Die Wärmeverluste, aufgrund welcher die Dialysierflüssigkeit abkühlt, während diese vom Heizbeutel 162 bis zum Einlass der Dialysierflüssigkeitskammer 303a des Dialysators 303 gepumpt wird, können auf unterschiedliche Weise in einer solchen temperaturbasierten Regelung oder Begrenzung der Förderrate der ersten Flusspumpe 159 (auch Dialysierflüssigkeitspumpe, siehe Fig. 1 oder Fig. 3) berücksichtigt werden. Es kann beispielsweise ein Rechenmodell für die Wärmebilanz erstellt werden. Das Rechenmodell berechnet insbesondere die Wärmeverluste an der Dialysierflüssigkeitszulaufleitung 104. Dies erfolgt beispielsweise in Abhängigkeit von der Förderrate der ersten Flusspumpe 159, den Eigenschaften der Dialysierflüssigkeitszulaufleitung 104, weiteren Randbedingungen, wie beispielsweise der Umgebungstemperatur, und/oder den in der Speichervorrichtung 500 für die Temperaturhistorie hinterlegten Temperaturwerten Tmax. Die Förderrate der ersten Flusspumpe 159 kann basierend auf dieser Berechnung derart bestimmt werden, dass die Temperatur an der vorbestimmten Stelle, beispielsweise am Einlass der Dialysierflüssigkeitskammer 303a des Dialysators 303, die maximal zulässige Temperatur einerseits nicht überschreitet und andererseits die Solltemperatur nicht wesentlich unterschreitet.

Um den Programmieraufwand solcher Rechenmodelle für die Wärmebilanz zu begrenzen, können in manchen Ausführungsformen der vorliegenden Erfindung unter Berücksichtigung der Abkühlung der in der Dialysierflüssigkeitszulaufleitung 104 strömenden Dialysierflüssigkeit empirisch in Versuchsreihen Zuordnungen der Werte
(i) der maximalen Temperatur an der vorbestimmten Stelle,
(ii) des maximal gemessenen Temperaturwerts an der Temperaturmessvorrichtung 450 und
(iii) der verwendeten Förderrate der Dialysierflüssigkeitspumpe
ermittelt worden und in der Software in einer Nachschlagetabelle hinterlegt worden sein.

In manchen Ausführungsformen der vorliegenden Erfindung wird für die Ermittlung des prominenten, z. B. maximalen Temperaturwerts innerhalb eines Messintervalls bei der Passage der Temperaturmessvorrichtung 450, hier des Ringsensors, über z. B. 15 Sekunden, die Temperatur der Behandlungsflüssigkeit mehrfach gemessen, um aus diesen gemessenen Temperaturwerten den prominenten, z. B. maximalen, Temperaturwert Tmax zu erheben. Dies wird für eine Reihe von aufeinanderfolgenden Messintervallen durchgeführt, von denen die entsprechend geförderten Teilvolumina der Behandlungsflüssigkeit, die in beispielsweise diesen 15 Sekunden gefördert wurden, bekannt sind.

Die Steuervorrichtung 150 ermittelt für die Einstellung der Förderrate der ersten Flusspumpe 159, hier der Dialysierflüssigkeitspumpe (in Fig. 2 nicht gezeigt), deren im Lichte der erhobenen Temperaturwerte maximal zulässige Förderrate und steuert sie dementsprechend. Das Ermitteln kann beispielsweise basierend auf einer Nachschlagetabelle für die maximal zulässige Temperatur an der vorbestimmten Stelle, die in der Speichervorrichtung hinterlegt sein kann, in Verbindung mit dem prominenten, hier maximalen, erhobenen Temperaturwert, welcher an der Temperaturmessvorrichtung 450 in einem bestimmten Messintervall gemessen wurde, erfolgen. Die Erfinder konnten zeigen, dass die maximale Temperatur der Dialysierflüssigkeit an der vorbestimmten Stelle mit der erfindungsgemäßen Regelung zuverlässig und mit ausreichender Genauigkeit eingehalten werden kann.

In manchen Ausführungsformen wird bei stehender erster Flusspumpe 159 in der Software ein "virtueller" Dialysierflüssigkeitsfluss von 5 ml/min angenommen und ebenfalls die Temperatur an der Temperaturmessvorrichtung 450 im Datenspeicher für die Temperaturhistorie gespeichert, um die Abkühlung berücksichtigen zu können.

Somit wird der Regelung der Heizleistung der Heizvorrichtung 162 anhand der mittels der Temperaturmessvorrichtung 450 gemessenen Temperaturwerte Tmax vorteilhaft zusätzlich eine temperaturbasierte Regelung oder Begrenzung der Förderrate der ersten Flusspumpe 159 hinzugefügt, um an der vorbestimmten Stelle eine maximale Temperatur nicht zu übersteigen und eine Solltemperatur nicht zu unterschreiten.

Alles hierin zur Heizvorrichtung 162, welche in der Dialysierflüssigkeitszulaufleitung 104 angeordnet ist, Ausgeführte gilt analog auch für Heizvorrichtungen 162a, welche in einer Substituatleitung 105, 107a, 109a (siehe hierzu die Beschreibung zur Fig. 1 und zur Fig. 3) angeordnet sein können. Eine vorbestimmte Stelle ist in diesen Ausführungsformen beispielsweise eine Zugabestelle 107, 109 für Dialysierflüssigkeit als Substituat in Prä- oder Postdilution in den extrakorporalen Blutkreislauf 300, die fördernde Pumpe ist demnach eine Substituatpumpe 111 (siehe Fig. 1 und Fig. 3).

**Fig. 3** zeigt schematisch vereinfacht einen Fluidleitungsaufbau einer Blutbehandlungsvorrichtung 100 in einer weiteren beispielhaften Ausführungsform.

Es wird hierin auf die Beschreibung der Fig. 1 Bezug genommen. Nachfolgend wird insbesondere auf die Unterschiede zur Fig. 1 eingegangen.

Die Blutbehandlungsvorrichtung 100 ist verbunden mit einem extrakorporalen Blutkreislauf 300, welcher zu einer Behandlung mittels Double-Needle-Zugangs, oder unter Verwendung z. B. eines zusätzlichen Y-Verbinders (Bezugszeichen Y) wie in Fig. 3 gezeigt mittels Single-Needle-Zugangs mit dem Gefäßsystem des nicht dargestellten Patienten Pa verbunden werden kann. Der extrakorporale Blutkreislauf 300 kann optional in Abschnitten hiervon in oder auf einer Blutkassette vorliegen.

Pumpen, Aktoren und/oder Ventile im Bereich des extrakorporalen Blutkreislaufs 300 sind mit der erfindungsgemäßen Blutbehandlungsvorrichtung 100 bzw. mit einer von dieser z. B. umfassten, erfindungsgemäßen Steuervorrichtung 150, die eine Regelvorrichtung sein kann, verbunden. Die hierin gemachten Ausführungen zur Steuervorrichtung 150, insbesondere zu Fig. 1 und Fig. 2, gelten analog auch für die Steuervorrichtung 150 der Fig. 3.

Analog zu Fig. 1 ist die optionale Blutpumpe 101 in oder an der arteriellen Leitung 301 vorgesehen. Die Substituatpumpe 111 ist in dieser Ausführungsform in oder an einer Substituatleitung 105 angeordnet und kann fluidisch mit der Dialysierflüssigkeitszulaufleitung 104 verbunden sein. Mittels der Substituatpumpe 111 kann Substituat per Prädilution, über ein Prädilutionsventil 107, oder per Postdilution, über ein Postdilutionsventil 109, über zugehörige Leitungen 107a bzw. 109a in Leitungsabschnitte, beispielsweise in den arteriellen Leitungsabschnitt 301 bzw. in den venösen Leitungsabschnitt 305 (hier zwischen einer Blutkammer 303b eines Blutfilters 303 und einem Blasenfänger 329, hier einer venösen Luftabscheidekammer) des Blutschlauchsatzes 300 eingebracht werden.

Die Anordnung der Fig. 3 umfasst ferner optional ein Ventil V24, welches in der Dialysierflüssigkeitszulaufleitung 104, stromaufwärts des Blutfilters, jedoch stromab eines ebenfalls optionalen ersten Drucksensors PS5 angeordnet ist. Sie umfasst weiter ein optionales Ventil V25, welches in der Dialysatablaufleitung 102, stromabwärts des Blutfilters 303, jedoch stromauf des optionalen Drucksensors PS4, angeordnet ist.

Die Anordnung der Fig. 3 umfasst den Luftblasendetektor 315 zum Detektieren von Luft und/oder Blut, einen oder zwei Drucksensoren PS1 (stromauf der Blutpumpe 101) und PS2 (stromab der Blutpumpe 101, er misst den Druck stromauf des Blutfilters 303 ("prä-Hämofilter")), z. B. an den in Fig. 3 gezeigten Stellen. Weitere Drucksensoren können vorgesehen sein, z. B. der Drucksensor PS3 stromab des venösen Blasenfängers 329.

Eine optionale Single-Needle-Kammer 317 kommt in Fig. 3 als Puffer- und/oder Ausgleichsbehälter bei einem Single-Needle-Verfahren zum Einsatz, bei welchem der Patient mittels nur einer Konnektionsnadel mit den zwei Blutleitungen 301, 305 mit dem extrakorporalen Blutkreislauf 300 verbunden ist.

Eine Zugabestelle 325 für Heparin kann optional vorgesehen sein.

Auf der Maschinenseite, links in Fig. 3, ist eine Anmischvorrichtung 163 gezeigt, welche aus den Behältern A (für A-Konzentrat über die Konzentratversorgung 166) und B (für B Konzentrat über die Konzentratversorgung 168) eine vorbestimmte Mischung für die jeweilige Lösung zur Verwendung durch die Blutbehandlungsvorrichtung 100 bereitstellt. Die Lösung enthält Wasser aus einer Wasserquelle 155 (online, z. B. als Umkehrosmosewasser oder aus Beuteln), welches z. B. in der Heizvorrichtung 162 angewärmt wird.

Eine Pumpe 171, welche als Konzentratpumpe oder Natriumpumpe bezeichnet werden kann, ist mit der Anmischvorrichtung 163 und einer Quelle mit Natrium, etwa dem Behälter A, fluidisch verbunden und/oder fördert hieraus. Eine optionale Pumpe 173, welche dem Behälter B, etwa für Bicarbonat, zugeordnet ist, ist zu erkennen.

Weiter ist in Fig. 3 ein Abfluss 153 für das Effluent zu erkennen. Ein optionaler Wärmetauscher 157 und die erste Flusspumpe 159, die zur Entgasung geeignet ist, ergänzen die gezeigte Anordnung.

Der Drucksensor PS4 stromab des Blutfilters 303 auf der Wasserseite, jedoch vorzugsweise stromauf einer Ultrafiltrationspumpe 131 in der Dialysatablaufleitung 102 kann zum Messen des Filtratdrucks oder Membrandrucks des Blutfilters 303 vorgesehen sein.

Blut, das den Blutfilter 303 verlässt, durchströmt einen optionalen venösen Blasenfänger 329, welcher eine Entlüftungseinrichtung 318 aufweisen und mit dem Drucksensor PS3 in Fluidverbindung stehen kann.

Mittels der Vorrichtung zur Online-Mischung der Dialysierflüssigkeit ist eine Variation deren Natriumgehalts, gesteuert durch die Steuervorrichtung 150, in bestimmten Grenzen möglich. Hierzu können insbesondere die mittels Leitfähigkeitssensoren 163a, 163b ermittelten Messwerte einbezogen werden. Sollte sich dabei eine Anpassung des Natriumgehalts der Dialysierflüssigkeit (Natriumkonzentration) oder des Substituats als erforderlich oder gewünscht ergeben, so kann dies durch Anpassen der Förderrate der Natriumpumpe 171 erfolgen.

Darüber hinaus umfasst die Blutbehandlungsvorrichtung 100 Mittel zur Förderung von frischer Dialysierflüssigkeit sowie von Dialysat. Ein erstes Ventil kann zwischen der ersten Flusspumpe 159 und dem Blutfilter 303 vorgesehen sein, welches eingangsseitig den Zulauf zum Blutfilter 303 öffnet bzw. schließt. Die zweite, optionale Flusspumpe 169 ist z. B. stromabwärts des Blutfilters 303 vorgesehen, und fördert Dialysat zum Abfluss 153. Ein zweites Ventil kann zwischen dem Blutfilter 303 und der zweiten Flusspumpe 169 vorgesehen sein, welches ausgangsseitig den Ablauf öffnet bzw. schließt. Weiterhin umfasst die Blutbehandlungsvorrichtung 100 optional eine Vorrichtung 161 zur Bilanzierung des in den Dialysator 303 ein- und ausströmenden Flusses auf der Maschinenseite. Die Vorrichtung 161 zur Bilanzierung ist vorzugsweise in einem Leitungsbereich zwischen der ersten Flusspumpe 159 und der zweiten Flusspumpe 169 angeordnet.

Die Blutbehandlungsvorrichtung 100 umfasst ferner Mittel zum exakten Entfernen eines durch den Anwender und/oder durch die Steuervorrichtung 150 vorgegebenen Flüssigkeitsvolumens aus dem bilanzierten Kreislauf wie die Ultrafiltrationspumpe 131.

Sensoren wie die optionalen Leitfähigkeitssensoren 163a, 163b dienen der Bestimmung der, in manchen Ausführungsformen temperaturkompensierten, Leitfähigkeit sowie des Flüssigkeitsstroms stromauf und stromab des Dialysators 303.

Temperatursensoren 165a, 165b können einzeln oder zu mehreren vorgesehen sein. Von ihnen gelieferte Temperaturwerte können erfindungsgemäß zum Ermitteln einer temperaturkompensierten Leitfähigkeit genutzt werden.

Ein Anschluss für eine optionale Drucklufteinrichtung, beispielsweise in Form eines Kompressors, kann ergänzend maschinenseitig stromauf des Blutfilters 303 und/oder an anderer Stelle vorgesehen sein (hier nicht gezeigt).

Ein Leckagesensor 167 ist optional vorgesehen. Er kann alternativ auch an anderer Stelle vorgesehen sein.

Weitere Flusspumpen, ergänzend oder alternativ zu z. B. jener mit dem Bezugszeichen 169, können ebenfalls vorgesehen sein.

Eine Reihe von optionalen Ventilen ist in Fig. 3 jeweils mit V bezeichnet. Bypassventile sind mit VB bezeichnet.

Basierend auf den Messwerten der vorgenannten, optionalen Sensoren ermittelt in einigen Ausführungsformen die Steuervorrichtung 150 die Elektrolyt- und/oder Flüssigkeitsbilanz.

Ein Filter F1 dient hier exemplarisch dazu, mittels der Anmischvorrichtung 163 selbst unter Nutzung von nicht-reinem Wasser ausreichend reine Dialysierflüssigkeit zu erzeugen, welche anschließend, z. B. im Gegenstromprinzip, durch den Blutfilter 303 strömt.

Ein Filter F2 dient hier exemplarisch dazu, aus der ausreichend reinen Dialysierflüssigkeit, welche den ersten Filter F1 verlässt, durch Filtern von z. B. pyrogenen Stoffen steriles oder ausreichend gefiltertes Substituat zu generieren, welches bedenkenlos dem extrakorporal strömenden Blut des Patienten und damit letztlich dem Körper des Patienten zugeführt werden kann.

Die Filter F1 und F2 können in Serie geschaltet vorgesehen sein.

Die Blutbehandlungsvorrichtung 100 ist in Fig. 3 zwar optional als Vorrichtung zur Hämo(dia)filtration gezeigt. Hämodialysevorrichtungen fallen jedoch ebenfalls unter die vorliegende Erfindung, obgleich nicht eigens mittels Figur dargestellt.

Die vorliegende Erfindung ist nicht auf die vorstehend beschriebenen Ausführungsformen beschränkt, diese dienen lediglich der Veranschaulichung.

Die in den Figuren gezeigten Pfeile oder Pfeilspitzen geben allgemein jeweils die Strömungsrichtung an.

Heizvorrichtungen mit zugehörigen Temperaturmessvorrichtungen, wie sie in Fig. 1 oder 2 gezeigt und hierin offenbart sind, können in der Blutbehandlungsvorrichtung 100 der Fig. 3 vorgesehen sein.

### Bezugszeichenliste

- 100: Blutbehandlungsvorrichtung

- 101: Blutpumpe
- 102: Dialysatablaufleitung
- 104: Dialysierflüssigkeitszulaufleitung
- 105: Substituatleitung
- 107: Prädilutionsventil
- 107a: zum Prädilutionsventil gehörige Leitung
- 109: Postdilutionsventil
- 109a: zum Postdilutionsventil gehörige Leitung
- 111: Substituatpumpe
- 131: Ultrafiltrationspumpe

- 150: Steuervorrichtung

- 153: Abfluss
- 155: Wasserquelle
- 157: Wärmetauscher
- 159: erste Flusspumpe
- 161: Vorrichtung zur Bilanzierung
- 162: Heizvorrichtung
- 162a: Heizvorrichtung
- 162b: beheizter Bereich
- 162c: nicht beheizter Bereich
- 162d: Volumen im Heizbeutel stromabwärts des Ringsensors
- 163: Anmischvorrichtung
- 163a: Leitfähigkeitssensor
- 163b: Leitfähigkeitssensor
- 165a: Temperatursensor
- 165b: Temperatursensor
- 166: Konzentratversorgung
- 167: Leckagesensor
- 168: Konzentratversorgung
- 169: zweite Flusspumpe
- 171: Pumpe, Natriumpumpe
- 173: Pumpe, Bicarbonatpumpe

- 300: extrakorporaler Blutschlauchsatz oder Blutkreislauf
- 301: erste Leitung (arterieller Leitungsabschnitt)
- 302: arterielle Patientenschlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: venöse Patientenschlauchklemme
- 315: Luftblasendetektor; Air Bubble Detector; ABD
- 317: Single-Needle-Kammer
- 318: Entlüftungseinrichtung
- 325: Zugabestelle für Heparin
- 329: (venöser) Blasenfänger

- 400: Effluentbeutel; Filtratbeutel
- 401: Dialysierflüssigkeitsquelle; Beutel mit Dialysierflüssigkeit
- 403: Substituatquelle; Beutel mit Substituat

- 450: Temperaturmessvorrichtung; Temperatursensor
- 500: Speichervorrichtung

- A: Behälter; A-Konzentrat; Natrium
- B: Behälter; B-Konzentrat; Bicarbonat

- DV: gefördertes Dialysierflüssigkeitsvolumen
- F1: Filter
- F2: Filter
- Pa: Patient
- PS1: arterieller Drucksensor (optional)
- PS2: arterieller Drucksensor (optional)
- PS3: Drucksensor (optional)
- PS4: Drucksensor zum Messen des Filtratdrucks (optional)
- PS5: Drucksensor zum Messen des Drucks in der Dialysierflüssigkeitszulaufleitung

- Tmax: maximale, im Messintervall gemessene Temperatur

- t: Zeitachse

- V: Ventile
- V24: Ventil
- V25: Ventil
- VB: Bypassventile

- W: Waage

- Y: Y-Verbinder

## Patentansprüche

1. Blutbehandlungsvorrichtung (100) mit
- optional: einer Blutpumpe (101),
- einer Fluidleitung (104, 107a, 109a) oder einer Verbindungsstelle zum Verbinden mit einer Fluidleitung (104, 107a, 109a),
- einer Pumpe, konfiguriert zum Fördern von Behandlungsflüssigkeit durch die Fluidleitung (104, 107a, 109a),
- einer Heizvorrichtung (162, 162a), konfiguriert zum Erwärmen der Behandlungsflüssigkeit innerhalb der Fluidleitung (104, 107a, 109a) und/oder innerhalb der Heizvorrichtung (162, 162a),
- einer Temperaturmessvorrichtung (450), konfiguriert zum Erheben, z. B. Messen oder Ermitteln, der Temperatur der Behandlungsflüssigkeit mittels wenigstens eines Temperatursensors, wenn die Behandlungsflüssigkeit in der Fluidleitung (104, 107a, 109a) oder in der Heizvorrichtung (162, 162a) vorliegt,
- einer Speichervorrichtung (500) zum Speichern von Beträgen von Temperaturwerten,
- einer Steuervorrichtung (150) zum Steuern oder Regeln der Pumpe konfiguriert zum Fördern von Behandlungsflüssigkeit,
wobei die Steuervorrichtung (150) programmiert ist,
- um die Temperaturmessvorrichtung (450) zu veranlassen, zu einer Vielzahl von Messzeitpunkten, welche verschiedenen Messintervallen zugeordnet sind, jeweils einen Temperaturwert zu messen, und zu jedem der Messintervalle aus den während des Messintervalls gemessenen Temperaturwerten jeweils einen prominenten, z. B. den maximalen, Temperaturwert zu erheben;
- um die Vielzahl der so erhobenen Temperaturwerte in die/der Speichervorrichtung (500) zu speichern;
- um die gespeicherten, erhobenen Temperaturwerte aus der Speichervorrichtung (500) auszulesen;
- um einen voreingestellten oder vorbestimmten Betrag der Temperatur, welche die Behandlungsflüssigkeit an einer vorbestimmten Stelle innerhalb der Fluidleitung stromab der Heizvorrichtung (162) als Maximalwert betragen darf, z. B. aus der Speichervorrichtung (500), auszulesen;
- um eine Abkühlung der Behandlungsflüssigkeit bei ihrer Passage zur vorbestimmten Stelle, z. B. zwischen der Temperaturmessvorrichtung (450) und der vorbestimmten Stelle, zumindest näherungsweise, zu bestimmen oder anzunehmen;
- um zu ermitteln, z. B. zu berechnen, wie hoch die Förderrate der Pumpe maximal sein darf, damit die sich bereits stromab der Heizvorrichtung (162, 162a) in der Fluidleitung befindende Behandlungsflüssigkeit, deren erhobene Temperaturwerte, z. B. aus der Speichervorrichtung (500), ausgelesen wurden, mit einer Temperatur kleiner oder gleich des Maximalwerts die vorbestimmte Stelle erreicht, und
- um die Förderrate der Pumpe zum Fördern von Behandlungsflüssigkeit derart zu begrenzen oder anderweitig zu steuern oder regeln, dass die Temperatur, welche die von ihr geförderte Behandlungsflüssigkeit, wenn sie an die vorbestimmte Stelle gefördert ist, aufweist, einen vorbestimmten Temperaturwert nicht überschreitet.

2. Blutbehandlungsvorrichtung (100) nach Anspruch 1, wobei die Pumpe zum Fördern der Behandlungsflüssigkeit eine Dialysierflüssigkeitspumpe (159) ist, angeordnet zum Fördern der Behandlungsflüssigkeit als Dialysierflüssigkeit in eine Dialysierflüssigkeitskammer (303a) eines Blutfilters (303), welcher die Dialysierflüssigkeitskammer (303a) und, von dieser mittels einer Membran (303c) getrennt, eine Blutkammer (303b) aufweist.

3. Blutbehandlungsvorrichtung (100) nach Anspruch 1, wobei die Pumpe eine Substituatpumpe (111) ist.

4. Blutbehandlungsvorrichtung (100) nach Anspruch 1 oder 2, wobei die vorbestimmte Stelle die Dialysierflüssigkeitskammer (303a) oder ein Einlass in den Blutfilter (303) ist.

5. Blutbehandlungsvorrichtung (100) nach Anspruch 1, 2 oder 3, wobei die vorbestimmte Stelle eine Zugabestelle (107, 109) für Behandlungsflüssigkeit als Substituat in den extrakorporalen Blutkreislauf (300) ist.

6. Blutbehandlungsvorrichtung (100) nach einem der vorangegangenen Ansprüche, wobei die Länge bzw. Dauer der Messintervalle konstant ist und in der Speichervorrichtung (500) gespeichert ist.

7. Blutbehandlungsvorrichtung (100) nach einem der vorangegangenen Ansprüche, wobei die Beträge der in den Messintervallen jeweils geförderten Mengen an Behandlungsflüssigkeit gespeichert werden.

8. Blutbehandlungsvorrichtung (100) nach einem der vorangegangenen Ansprüche, wobei die Beträge der in den Messintervallen jeweils geförderten Mengen an Behandlungsflüssigkeit gemeinsam mit dem erhobenen Temperaturwert und einer Bezeichnung zum Identifizieren des zugehörigen Messintervalls, für welches der Temperaturwert jeweils erhoben wurde, gespeichert werden.

9. Blutbehandlungsvorrichtung (100) nach einem der vorangegangenen Ansprüche, wobei die Abkühlung der Behandlungsflüssigkeit bei ihrer Passage zwischen der Temperaturmessvorrichtung (450) und der vorbestimmten Stelle anhand von einer oder mehreren Nachschlagetabellen und/oder durch Berechnung ermittelt oder zumindest näherungsweise, bestimmt oder angenommen werden.

10. Blutbehandlungsvorrichtung (100) nach einem der vorangegangenen Ansprüche, wobei die Speichervorrichtung (500) ein Ringpuffer oder ein Ringspeicher ist oder einen solchen aufweist, in welchem die erhobenen Temperaturwerte gespeichert oder zu speichern sind.

11. Blutbehandlungsvorrichtung (100) nach einem der vorangegangenen Ansprüche, wobei die Steuervorrichtung (150) programmiert ist, um die erhobenen Temperaturwerte, insbesondere nach ihrer Speicherung, für Zeiträume, in welchen die Pumpe zum Fördern von Behandlungsflüssigkeit nicht fördert, mit einem Korrekturfaktor nach unten zu korrigieren.

## Claims

1. A blood treatment apparatus (100) comprising:
- optionally: a blood pump (101),
- a fluid line (104, 107a, 109a) or a connection site for the connection to a fluid line (104, 107a, 109a),
- a pump, configured to convey treatment liquid through the fluid line (104, 107a, 109a),
- a heating device (162, 162a), configured to heat the treatment liquid within the fluid line (104, 107a, 109a) and/or within the heating device (162, 162a),
- a temperature measuring device (450), configured to collect, e.g. to measure or determine, the temperature of the treatment liquid, using at least one temperature sensor when the treatment liquid is present in the fluid line (104, 107a, 109a) or in the heating device (162, 162a),
- a storage device (500) for storing amounts of temperature values,
- a control device (150) for controlling or regulating the pump configured for conveying treatment liquid,
wherein the control device (150) is programmed:
- to prompt that the temperature measuring device (450) respectively measures a temperature value at several measurement times corresponding with different measurement intervals, and, for each of the measurement intervals, respectively to collect a notable temperature value, e.g. the maximum temperature value, from among the temperature values measured during the measurement interval;
- to store the plurality of temperature values thus collected in the storage device (500);
- to read out the stored, collected temperature values from the storage device (500);
- to read out, e.g. from the storage device (500), a preset or predetermined amount of the temperature, which the treatment liquid may amount, as a maximum value at a predetermined site within the fluid line downstream of the heating device (162);
- to determine or assume, at least approximately, a cooling of the treatment liquid during its passage towards the predetermined site, e.g. between the temperature measuring device (450) and the predetermined site;
- to determine, e.g. to calculate, how high the maximum conveyance rate of the pump may be, so that the treatment liquid already present downstream of the heating device (162, 162a) in the fluid line, whose collected temperatures values have been read out, e.g. from the storage device (500), reaches the predetermined site at a temperature lower than or equal to the maximum value, and
- to limit, or to control or regulate in another way, the pump's conveyance rate for conveying treatment liquid so that the temperature of the treatment liquid conveyed by it does not exceed a predetermined temperature value when the treatment liquid is conveyed to the predetermined site.

2. The blood treatment apparatus (100) according to claim 1, wherein the pump for conveying the treatment liquid is a dialysis liquid pump (159) arranged to convey the treatment liquid as dialysis liquid into a dialysis liquid chamber (303a) of a blood filter (303) comprising the dialysis liquid chamber (303a) and a blood chamber (303b), separated therefrom via a membrane (303c).

3. The blood treatment apparatus (100) according to claim 1, wherein the pump is a substitute pump (111).

4. The blood treatment apparatus (100) according to claim 1 or 2, wherein the predetermined site is the dialysis liquid chamber (303a) or an inlet into the blood filter (303).

5. The blood treatment apparatus (100) according to claim 1, 2 or 3, wherein the predetermined site is an addition site (107, 109) for treatment liquid as a substitute in the extracorporeal blood circuit (300).

6. The blood treatment apparatus (100) according to any one of the preceding claims, wherein the length or duration of the measurement intervals is constant and is stored in the storage device (500).

7. The blood treatment apparatus (100) according to any one of the preceding claims, wherein the amounts of treatment liquid conveyed in each of the measurement intervals are stored.

8. The blood treatment apparatus (100) according to any one of the preceding claims, wherein the amounts of treatment liquid conveyed in each measurement intervals are stored together with the collected temperature value and a designation identifying the corresponding measurement interval for which the temperature value was respectively collected.

9. The blood treatment apparatus (100) according to any one of the preceding claims, wherein the cooling of the treatment liquid during its passage between the temperature measuring device (450) and the predetermined site is determined, or at least approximately, ascertained or assumed using one or more look-up tables and/or by calculation.

10. The blood treatment apparatus (100) according to any one of the preceding claims, wherein the storage device (500) is, or comprises, a ring buffer or a ring storage, in which the collected temperature values are stored or are to be stored.

11. The blood treatment apparatus (100) according to any one of the preceding claims, wherein the control device (150) is programmed to downwardly correct the collected temperature values, particularly after storage, using a correction factor for periods in which the pump does not convey the treatment liquid.

## Revendications

1. Un appareil de traitement du sang (100) comprenant :
- en option : une pompe à sang (101),
- un conduit de fluide (104, 107a, 109a) ou un point de raccordement pour le raccordement à un conduit de fluide (104, 107a, 109a),
- une pompe, configurée pour acheminer le liquide de traitement au travers du conduit de fluide (104, 107a, 109a),
- un dispositif de chauffage (162, 162a), configuré pour réchauffer le liquide de traitement à l'intérieur du conduit de fluide (104, 107a, 109a) et/ou à l'intérieur du dispositif de chauffage (162, 162a),
- un dispositif de mesure de température (450), configuré pour relever, par exemple pour mesurer ou déterminer, la température du liquide de traitement au moyen d'au moins un capteur de température, lorsque le liquide de traitement se trouve dans le conduit de fluide (104, 107a, 109a) ou dans le dispositif de chauffage (162, 162a),
- un dispositif d'enregistrement (500) pour enregistrer des quantités de valeurs de température,
- un dispositif de commande (150) pour commander ou réguler la pompe configurée pour acheminer le liquide de traitement,
où le dispositif de commande (150) est programmé pour :
- faire en sorte que le dispositif de mesure de température (450) mesure respectivement une valeur de température à plusieurs instants de mesure correspondant à différents intervalles de mesure, et, pour chacun des intervalles de mesure, relever respectivement une valeur de température notable, par exemple la valeur de température maximale, parmi les valeurs de températures mesurées pendant l'intervalle de mesure;
- enregistrer la multitude de valeurs de température ainsi relevées dans le dispositif d'enregistrement (500);
- lire les valeurs de température relevées enregistrées à partir du dispositif d'enregistrement (500);
- lire, par exemple à partir du dispositif d'enregistrement (500), une quantité prédéfinie ou prédéterminée de la température que le liquide de traitement peut atteindre comme valeur maximale à un emplacement prédéterminé à l'intérieur du conduit de fluide en aval du dispositif de chauffage (162);
- déterminer ou estimer, au moins approximativement, un refroidissement du liquide de traitement lors de son passage vers l'emplacement prédéterminé, par exemple entre le dispositif de mesure de température (450) et l'emplacement prédéterminé;
- déterminer, par exemple calculer, quel doit être le débit d'acheminement maximal de la pompe, afin que le liquide de traitement se trouvant déjà en aval du dispositif de chauffage (162, 162a) dans le conduit de fluide, dont les valeurs de température relevées ont été lues, par exemple à partir du dispositif d'enregistrement (500), atteigne l'emplacement prédéterminé à une température inférieure ou égale à la valeur maximale, et
- limiter, ou sinon contrôler ou réguler, le débit d'acheminement de la pompe afin d'acheminer du liquide de traitement de manière à ce que la température du liquide de traitement acheminé par celle-ci ne dépasse pas une valeur de température prédéterminée lorsque le liquide de traitement est acheminé vers l'emplacement prédéterminé.

2. L'appareil de traitement du sang (100) selon la première revendication, où la pompe servant à acheminer le liquide de traitement est une pompe à liquide de dialyse (159) agencée de façon à acheminer le liquide de traitement comme liquide de dialyse dans une chambre à liquide de dialyse (303a) d'un filtre sanguin (303), qui comporte la chambre à liquide de dialyse (303a) ainsi qu'une chambre à sang (303b) séparée de celle-ci via une membrane (303c).

3. L'appareil de traitement du sang (100) selon la première revendication, où la pompe est une pompe à substitut (111).

4. L'appareil de traitement du sang (100) selon la revendication 1 ou 2, où l'emplacement prédéterminé est la chambre à liquide de dialyse (303a) ou une entrée dans le filtre sanguin (303).

5. L'appareil de traitement du sang (100) selon la revendication 1, 2 ou 3, où l'emplacement prédéterminé est un point d'addition (107, 109) pour le liquide de traitement en tant que substitut dans le circuit sanguin extracorporel (300).

6. L'appareil de traitement du sang (100) selon l'une quelconque des revendications précédentes, où la longueur ou la durée des intervalles de mesure est constante et est enregistrée dans le dispositif d'enregistrement (500).

7. L'appareil de traitement du sang (100) selon l'une quelconque des revendications précédentes, où les quantités de liquide de traitement acheminées à chaque intervalle de mesure sont enregistrées.

8. L'appareil de traitement du sang (100) selon l'une quelconque des revendications précédentes, où les quantités de liquide de traitement acheminées à chaque intervalle de mesure sont enregistrées conjointement avec la valeur de température relevée et une désignation permettant d'identifier l'intervalle de mesure correspondant pour lequel la valeur de température a été respectivement relevée.

9. L'appareil de traitement du sang (100) selon l'une quelconque des revendications précédentes, où le refroidissement du liquide de traitement lors de son passage entre le dispositif de mesure de température (450) et l'emplacement prédéterminé est déterminé, ou au moins approximativement constaté ou estimé à l'aide d'une ou plusieurs tables de consultation et/ou par calcul.

10. L'appareil de traitement du sang (100) selon l'une quelconque des revendications précédentes, où le dispositif d'enregistrement (500) est ou comprend un tampon en anneau ou une mémoire en anneau dans laquelle les valeurs de température relevées sont enregistrées ou doivent être enregistrées.

11. L'appareil de traitement du sang (100) selon l'une quelconque des revendications précédentes, où le dispositif de commande (150) est programmé pour corriger à la baisse les valeurs de température relevées, notamment après leur enregistrement, au moyen d'un facteur de correction pendant des périodes durant lesquelles la pompe n'achemine pas le liquide de traitement.
